Europäisches Patentamt

European Patent Office

Office européen des brevets

(19)

(11) Publication number: **0 015 288**
**B1**

(12) **EUROPEAN PATENT SPECIFICATION**

(45) Date of publication of patent specification: **14.12.83**

(51) Int. Cl.³: **B 04 B 3/00,** B 04 B 15/02, F 16 J 15/36

(21) Application number: **79900886.7**

(22) Date of filing: **13.07.79**

(86) International application number:
PCT/US79/00490

(87) International publication number:
**WO 80/00227 21.02.80 Gazette 80/4**

(54) **CENTRIFUGE HAVING A ROTARY SEAL.**

(30) Priority: **17.07.78 US 925270**
**02.04.79 US 26292**

(43) Date of publication of application:
**17.09.80 Bulletin 80/19**

(45) Publication of the grant of the patent:
**14.12.83 Bulletin 83/50**

(84) Designated Contracting States:
**DE FR GB NL SE**

(56) References cited:
**US - A - 2 673 748**
**US - A - 2 858 149**
**US - A - 3 565 330**
**US - A - 3 652 183**
**US - A - 3 770 181**
**US - A - 3 801 142**

(73) Proprietor: **HAEMONETICS CORPORATION**
**400 Wood Road**
**Braintree Massachusetts 02184 (US)**

(72) Inventor: **LATHAM Jr., Allen**
**66 Malcolm Road**
**Jamaica Plain, MA 02130 (US)**

(74) Representative: **Holdcroft, James Gerald, Dr. et al,**
**Graham Watt & Co. Riverhead**
**Sevenoaks Kent TN13 2BN (GB)**

Courier Press, Leamington Spa, England.

## Centrifuge having a rotary seal

This invention relates to improved rotary centrifuge seal for use in centrifuges.

More particularly, the invention relates to a centrifuge for processing liquids and having a rotatable bowl, stationary elements, means respectively for admitting and discharging liquid to and from the centrifuge, and a dynamic seal between rotary and stationary parts of the centrifuge, the seal comprising a rotatable lower ring and a normally motionless upper ring.

Background Art

It is often necessary to provide an effective seal between rotatable and stationary parts of a fluid-processing centrifuge. For example, in a blood-processing centrifuge, the centrifuge bowl and associated parts often rotate while conduits making up the fluid inlet and outlet remain stationary. When processing blood, an effective rotary seal is required between these rotatable and stationary elements to ensure maintenance of sterile integrity of the blood and to prevent possible contamination of the surroundings when contaminated blood is being processed.

Such a rotary centrifuge seal must be extremely efficient; must minimize leakage of air into or out of the system; must maintain frictional heating at a minimum; must provide good dissipation of heat which may be generated; must be capable of tolerating moderate misalignment and vibration between rotating and stationary parts; and must minimize production of particulate contaminants which might be introduced into the blood being processed. In addition, it is important to have a seal which is low in manufacturing cost thereby making disposability of the entire blood pathway economically feasible after a single use.

A variety of types of rotary centrifuge seals have been developed in an attempt to maximize these qualities. Some examples of rotary centrifuge seals which have proven to be successful are described in U.S. Patents 3,409,213 and 3,565,330. In these patents, rotary seals are disclosed which are formed from a stationary rigid low friction member in contact with a moving rigid member to create a dynamic seal, and an elastomeric member which provides a resilient static seal as well as a modest closing force between the surfaces of the dynamic seal.

Another rotary seal suitable for use in blood-processing centrifuges is described in U.S. Patent 3,801,142. In this seal, a pair of seal elements having confronting annular fluid-tight sealing surfaces of non-corrodable material are provided. These are maintained in a rotatable but fluid-tight relationship by axial compression of a length of elastic tubing forming one of the fluid connections to these seal elements.

Another more recently developed rotary seal has been employed in a blood-processing centrifuge known as the B. T. Bowl which is marketed by Bellco, Mirandola, Italy. In this seal, a ceramic ring member is attached to rotatable elements of the centrifuge and a fixed graphite ring is attached to stationary centrifuge elements. These ring members are in sealing relationship with each other. Additionally, an elastomeric diaphragm is attached at one end to an adapter ring for the graphite ring and at the other end to a stationary part of the centrifuge. While this type of rotary seal helps reduce passage of wear particles into the blood pathway, it lacks adequate provision for assuring that wear particles will be ingested and expelled to the outside without entry into the blood pathway. The seal disclosed in Latham's U.S. Patent 3,565,330 likewise provides no assurance that particles will be prevented from reaching the blood pathway, and merely comprises a rotary lower ring contacting a non-rotating upper ring, both said rings having planar contacting surfaces.

U.S. patent specification 2,858,149 at first glance appears to show a seal closely akin to the seal taught herein. The prior seal comprises two seal rings one (the lower) of which may be grooved. In any event, whether or not a grooved seal ring is involved, one of the rings has a slightly conical surface contacting the other ring. The object allegedly achieved by this is that should foreign particles find their way between the rings, the line seal is not destroyed but is merely shifted radially. If grooves are provided in one seal ring, they serve as pockets to retain grit. The seal structure shown in this patent does not have any capability of positively discharging foreign matter to the exterior. Thus, this prior seal provides no significantly better safeguard against ingress of grit than the seal in the B T Bowl or the seal shown in US.—A 3,565,330.

It is the object of this invention to expel particles positively to the outside of the seal, thus away from the liquid pathway of a centrifuge.

The present invention is characterised in that radially inwardly of their area of sealing contact the rings are formed to provide a space therebetween wherein solid particulate matter generated at the area of contact is entrapped in use and caused to settle upon the lower ring which, by its rotation, sweeps the solid matter in a radially outward direction into the area of contact for ingestion therein and ultimate expulsion radially outwards from the seal.

In one embodiment, the space is provided by one or more recessed areas in the sealing surface of the normally motionless ring member so that an area of non-contact is formed contiguous to and radially inwardly of the area of contact. Alternative embodiments include seals having a normally motionless seal ring with an extension providing increased surface area for the dissipation of heat and/or for providing a temporary increase in torque in order to achieve breakaway in exceptional circumstances where the two seal rings have unusual adherence to each other.

The centrifuge rotary seal can optionally be provided with a number of other features to minimize generation of solid particulate matter or to prevent such matter which may be generated from contaminating fluid being processed. For example, clearances between rotating and stationary centrifuge parts can be generously sized so that any slight misalignment which occurs during operation does not result in contact between rotating and fixed elements. This eliminates, of course, many of the potential sites where solid particulate matter could be generated. Additionally, the normally motionless seal ring can be provided with a convex radius at its inside lower corner and/or with a downwardly expanding slightly conical surface on its inner wall which will also help to direct any particulate matter generated towards the area of contact between the two ring members. A cylindrical shield also can be positioned inside the rotary seal rings attached to the rotatable ring and projecting well above the plane of the rubbing surfaces as a further way of preventing relatively large particles from reaching the fluid pathway. It may also be desirable to provide the lower ring member with a smaller inside diameter than the top ring to provide a shoulder to catch any particulate matter generated.

Brief Description of Drawings

Fig. 1 is a cross-sectional elevational view of a blood-processing centrifuge having a rotary seal typical of the type used in the prior art;

Fig. 2 is an exploded cross-sectional view of the ring members in the rotary seal of Fig. 1 and illustrates possible trajectories of solid particles generated at areas of contact in such a seal;

Fig. 3 is a cross-sectional view of the ring members in one embodiment of an improved rotary centrifuge seal according to this invention;

Fig. 4 is an exploded view of the ring members shown in Fig. 3 illustrating the entrapment and redirection of solid particles generated at areas of contact between the ring members of this seal;

Fig. 5 is a cross-sectional view of another embodiment of an improved rotary centrifuge seal according to this invention;

Fig. 6 is a cross-sectional elevational view of a blood-processing centrifuge bowl incorporating an improved rotary seal according to this invention;

Fig. 7 is a plan view of the centrifuge bowl of Fig. 6;

Fig. 8 is a partial cross-sectional elevational view of one alternative embodiment of the improved rotary seal according to this invention;

tion;

Fig. 9 is a partial cross-sectional view of another alternative embodiment of the improved rotary seal according to this invention; and,

Fig. 10 is a cross-sectional view along line 10—10 in Fig. 9.

Best Mode for Carrying Out the Invention

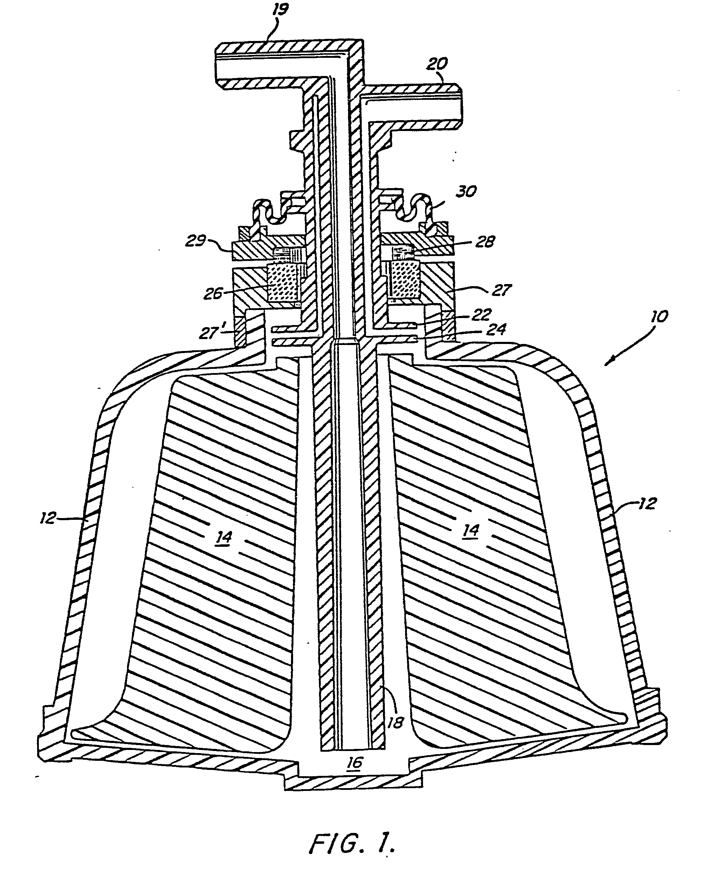

Fig. 1 illustrates a blood-processing centrifuge 10 incorporating a rotary centrifuge seal of a type typically used in the prior art. As can be seen, blood-processing centrifuge 10 has a centrifuge bowl 12 which rotates around a cylindrical core section 14 during operation to separate blood into its components. Whole blood is introduced into the central port 16 located at the bottom of centrifuge 10 through feed tube 18 which extends upwardly through the center of centrifuge 10 and then makes a 90° turn at its upper end to form inlet port 19 which can be connected to blood tubing (not shown). Feed tube 18 remains stationary during operation of centrifuge 10 whereas bowl 12 spins at high speed.

Peripheral port 20 is provided to allow separated blood components to flow out of centrifuge 10. Separated components are transported to peripheral port 20 through a channel formed between upper channel-defining member 22 and lower channel-defining member 24, both of which are attached to feed tube 18.

A rotary seal is formed from rotatable ceramic ring 26 attached by adapter rings 27 and 27' which in turn are attached to centrifuge bowl 12. Fixed graphite ring 28, having a larger inside diameter and a smaller outside diameter than ring 26, rests on top of ceramic ring 26. The upper surface of ceramic rotatable ring 26 is smooth and provides a sealing surface with a similar smooth and sealing surface on the bottom of ring 28. Therefore, contact between these respective surfaces forms a dynamic seal between rotatable and stationary elements of blood-processing centrifuge 10. A secondary seal is formed from elastomeric diaphragm 30, which is locked into a keyhole slot in feed tube 18 at one end and indirectly fixed to carbon ring 28 by an adhesive joint to adapter ring 29 at its other end.

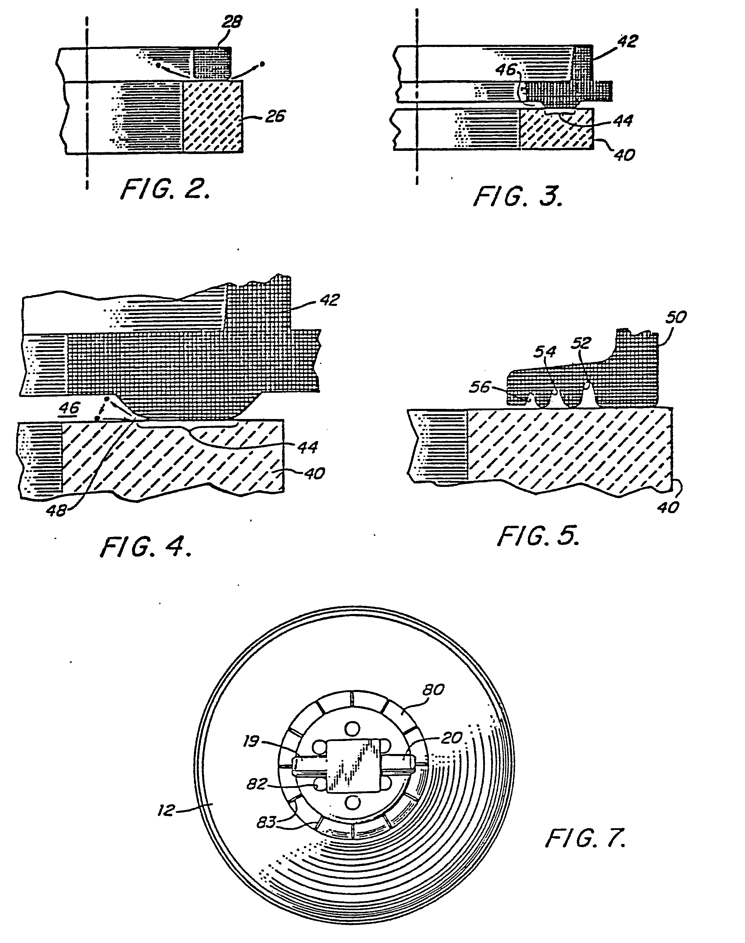

One serious problem encountered with a rotary centrifuge seal of the type illustrated in Fig. 1 is illustrated in Fig. 2. Therein, it can be seen that solid particles generated at contact areas between rotatable ceramic ring 26 and fixed graphite ring 28 during operation of centrifuge 10 can fly away from the dynamic seal. Thus, such particles can be projected in many directions and some of these particles can eventually find their way into blood components that have been processed. Thus, it is desirable to minimize the freedom of movement of such particles.

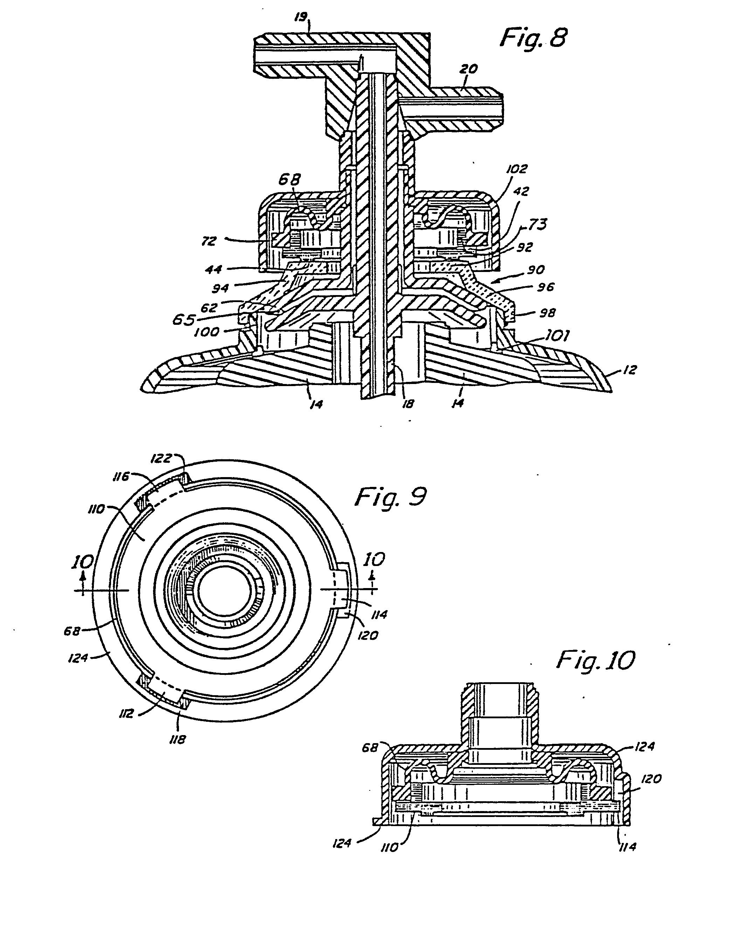

Two ring members of an improved rotary seal according to this invention are shown in Fig. 3. Thus, a rotatable ring member 40 is employed, as well as a normally motionless ring member 42, and there is an area of contact 44 between rings 40 and 42. The term "motionless" is used in conjunction with ring member 42 in preference to the term "fixed" because, although ring member 42 does remain stationary in normal operation, it is possible in certain embodiments for it to rotate slightly under certain exceptional circumstances prior to attaining a stationary position, as is described below, particularly with reference to Figs. 9 and 10.

Motionless ring member 42 has, however, been modified to provide increased protection against contamination of blood components with particulate matter generated by the rotary seal. This is achieved by providing an area of non-contact 46 which is located radially inwardly from the area of contact 44. This area of non-contact 46 can be formed by making a continuous recessed portion in the sealing face of ring 42 which is adjacent to the area of contact 44. A typical clearance between the sealing faces of ring members 40 and 42 at the area of non-contact 46 is 0.005 inches (0.13 mm).

The advantages of the rotary seal as shown in Fig. 3 are more specifically illustrated in Fig. 4. Therein, it can be seen that the area of non-contact 46 serves as a means for entrapping solid particles generated in the area of contact 44. One particle is shown for illustration, and this particle is first deflected by the recessed portion in the sealing surface of ring 42 and eventually settles upon the surface of rotatable ring member 40. Since ring member 40 is rotating at high speed during operation of the centrifuge, rotational velocity is imparted to the particle resting on its surface which causes the particle to be centrifugally conveyed back to the area of contact 44. Preferably, the surface of this recessed area is joined to the seal contact area by a section of conical surface 48 which slopes gradually toward the contact surface thus providing easy entry of particles into the region of contact 44. The particle is then ingested in the area of contact 44 and ground to fine particles which are expelled at the outer surface of the dynamic seal where they cannot find their way into blood components which are being processed.

Fig. 5 illustrates an alternative embodiment of an improved rotary centrifuge seal according to this invention. Once again, the seal has a rotatable lower ring 40. It also has a motionless seal ring 50 which has another configuration adjacent to its sealing surface to entrap particles and redirect them back to the area of contact between the ring members. Thus, a series of decreasingly shallower recessed portions 52, 54 and 56 serve a similar purpose to the single recessed portion shown in Fig. 3.

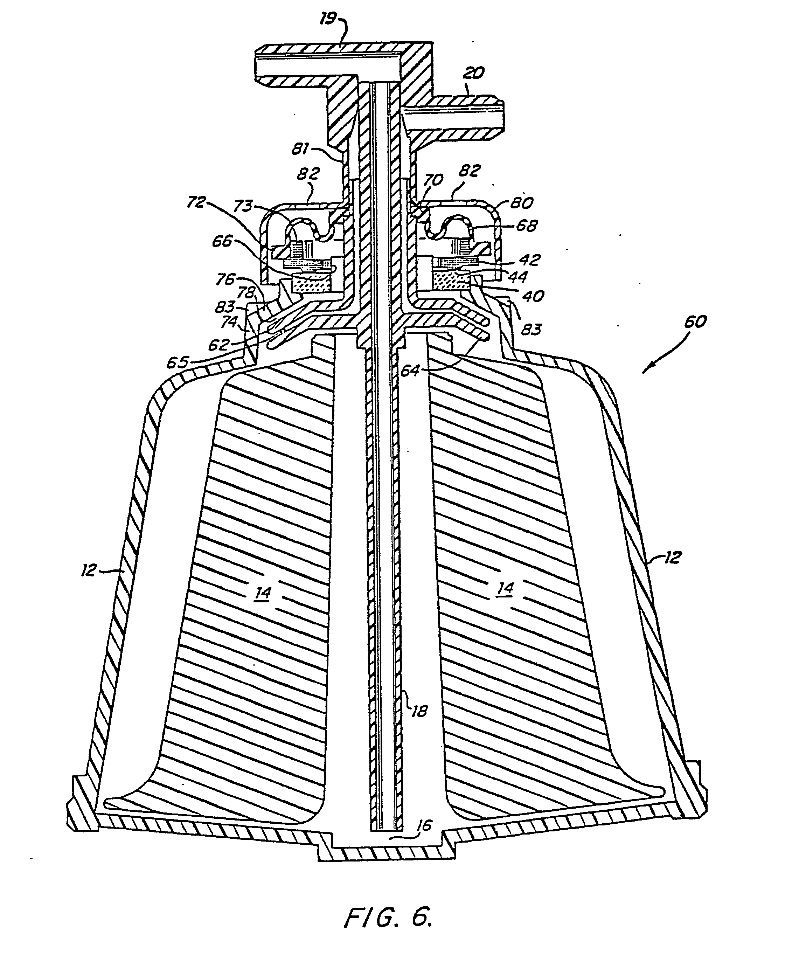

Fig. 6 illustrates a blood-processing centrifuge 60 similar but not identical to that of Fig. 1. Similar elements have been given similar numerals for purposes of convenience. Modified centrifuge 60 shown in Fig. 6 operates in a similar manner to centrifuge 10 shown in Fig. 1, except that it has an improved design to minimize particle generation and to entrap and redirect any particles which are generated.

Centrifuge 60 contains an improved rotary seal of the type illustrated in Figs. 3 and 4. In operation, this rotary seal prevents escape of sterilized gas, such as air, $CO_2$, etc., contained within the closed system and also prevents contamination of the sterilized gas by the external atmosphere.

This improved seal serves to entrap any particles on the blood pathway side generated between the sealing surfaces of lower ceramic rotatable ring 40 and upper graphite ring 42. As discussed above, any particles generated settle upon the shoulder of ring 40 and are then centrifugally conveyed towards the area of contact 44 between rings 40 and 42 where they are ingested, ground to extremely fine particles, and expelled outwardly from the seal so that they do not become entrained in blood being processed.

The rotary seal shown has several additional improvements. One of these is an internal rotating shield 66 which is attached to the inside wall of rotatable ring 40. Shield 66 can be formed from a variety of materials, but a material such as aluminium is preferred because of its light weight and corrosion-resistant properties. Shield 66 adds one more means for preventing solid particles which may be generated from entering blood components being processed.

Resilient diaphragm 68, which can be formed from an elastomeric material, has a lock configuration 70 at its upper end which fits snugly into a keyhole positioned in the upper section of upper channel-defining member 62. Its lower end 72 is designed so that it has to be stretched before it can be slipped over the vertical support 73 on ring member 42; therefore, no adhesive is usually necessary to hold diaphragm 68 over support 73. Adhesives can be used but are usually undesirable since they may run out from the areas in which they are applied thereby providing another possible contaminant within the centrifuge.

The shape and positioning of the respective elements forming the effluent channel for blood components is also improved in centrifuge 60. As shown, the improved rotary seal is positioned a considerable distance away from blood component effluent channel by means of vertical bowl section 74 and inclined bowl section 76. This provides sufficient distance between exiting blood components and the seal rings so that any heat generated at the seal rings will not be in close thermal contact with the blood components. Space 78 provides additional thermal insulation between exiting blood components and the rotary seal.

Both upper and lower channel-defining members 62 and 64 also have an extended diameter. The extended diameter serves to reduce the possibility that blood flowing into central port 16 will flow between inlet tube 18 and cylindrical core 14 thereby by-passing the separation zone in centrifuge 60. The extended diameter channel-defining members also provide more heat dissipation area in the area of the rotary seal.

Upper channel-defining member 62 is secured at its top end by a slip fit into vertical section 81 of the upper portion of the centrifuge structure. Lower channel-defining member 64 is integrally attached to the upper portion of inlet tube 18, and inlet tube 18 can be joined by an ultrasonic weld to the inner wall of inlet port 19. The proper distance between members 62 and 64 is maintained by protuberances 65.

To further minimize particle generation, generous clearances are provided between rotatable and stationary portions of centrifuge 60. Such generous clearances are particularly desirable: (1) between core 14 and feed tube 18; (2) between shield 66 and member 62; and between shield 66 and fixed graphite ring 42.

Centrifuge 60 is also provided with an external non-rotatable shield 80 which is part of an integral tube section 81. Shield 80 serves to protect the rotary seal from any blows, accidental or deliberate. Such a blow could open the seal thereby destroying the sterility of the system.

To enhance dissipation of heat generated by friction between the dynamic seal faces, air circulation ports 82 in shield 80 and small vanes 83 on rotating bowl 12 are provided, as illustrated. Vanes 83 can be seen more clearly in Fig. 7 and are appropriately sized to obtain good air circulation through the rotary seal without the concomittant disadvantage of creating excessive noise as bowl 12 rotates at high speed.

Fig. 8 illustrates an alternative embodiment of the improved rotary seal shown in a centrifuge similar to that of Fig. 6. Elements which are the same have been numbered consistently with elements of Fig. 6. Rotatable ring member 90 has a more complex shape than in previous embodiments, and includes a horizontally positioned ring portion 92, a steeply downwardly extending portion 94, a less steeply downwardly extending portion 96, and finally a vertical portion 98. Vertical portion 98 can be bonded to a mating surface 100 provided at the top of centrifuge bowl 12. The dynamic seal in this embodiment is formed at the area of contact 44 between the bottom sealing surface of the motionless graphite ring 42 and the upper sealing surface of the horizontal ring portion of ring member 90, which is similar to the seal formed by previous embodiments.

Ring member 90 can be formed from a good heat-conducting material, such as a metal, and thus can serve to conduct heat generated in operation away from the area of contact between ring members 42 and 90 to the extended surface areas of ring member 90 where such heat can be dissipated to air currents created as the centrifuge rotates. This prevents the build-up of heat which could result in a temperature rise with concomitant damage to blood components. Because of the excellent heat dissipation obtained through conduction along ring member 90 and subsequent convection to air currents, outer shield 102 need not be provided with air ports for cooling.

As shown, portion 96 of ring member 90 is resting upon upper channel defining member 62. This is the position of these elements when centrifuge 60 stands alone. However, when centrifuge 60 is locked into a chuck of permanent blood-processing apparatus, stationary components of centrifuge 60 are depressed resulting in a separation of member 62 from portion 96.

During normal operating conditions, the improved seal of this invention operates with unusual freedom from noise. This is at least partly due to the fact that the normal torque required to overcome the frictional resistance between the motionless and rotatable ring members is transmitted through the elastomeric diaphragm which forms a secondary seal without any direct physical contact between the motionless ring member and any of the other hard components of the assembly.

Under very exceptional circumstances, the break-away torque can be greater than the torque capacity of elastomeric diaphragm 68. An example of such exceptional circumstances would be the flooding of the seal with blood followed by a period of non-use sufficient to allow the blood to congeal or form a bond between the normally motionless ring member and the rotatable ring member. This potential problem can be overcome by providing slight modifications to the seal to enable it to momentarily produce the breakaway torque required at start-up without destruction of the seal.

One modification which allows the seal to withstand a higher than normal torque until breakaway is achieved is illustrated in Figs. 9 and 10. In this embodiment, motionless ring element 110 is provided with three equidistant tabs 112, 114, 116 on its outer periphery. Tabs 112, 114 and 116 extend into complementary recessed areas 118, 120 and 122, respectively, located on the inner surface of outer shield 124. In normal operation, tabs 112, 114 and 116 do not contact shield 124, which avoids wear and the generation of undesirable noise caused by contact between hard components. However, during an exceptional circumstance, where a high breakaway torque is required which exceeds that which can be tolerated by elastomeric diaphragm 68, normally motionless ring member 110 can rotate just slightly so that tabs 112, 114 and 116 contact the forward edge of recesses 118, 120 and 122, respectively. After such contact is established, torque builds up rapidly until breakaway occurs, after which ring member 110 is returned by the elastic return action of diaphragm 68 to a position where tabs 112, 114 and 116 are once again free floating within their respective recessed portions 118, 120 and 122.

A significant advantage of the improved rotary seal of this invention is the extremely quiet operation which can be achieved. There are several reasons for this, and some of these can be illustrated with reference to Figures 8, 9 and 10. As can be seen in Figure 8, resilient diaphragm 68 terminates in a flange 72 which extends beyond the edge of non-rotatable ring member 42. Thus, if misalignment between rotating and non-rotating parts in the centrifuge occurs, contact is made only between flange 72 and stationary shield 102. Since flange 72 is formed from resilient material, such as an elastomer, there is no significant noise generated by such contact. The only time that there is contact between hard non-rotating and hard rotating elements is in the extreme condition where momentary breakaway torque must be generated by contact of tabs 112, 114 and 116 with the leading edges of recesses 118, 120 and 122, respectively. As soon as the breakaway torque has been achieved, resilient diaphragm 68 returns tabs 112, 114 and 116 to their floating positions within their respective recesses. It should be noted that the inner circumferential wall members of recesses 118, 120 and 122 are spaced sufficiently far from the outer wall surfaces of tabs 112, 114 and 116 so that there is no contact therebetween. Other working clearances are also relatively large so that no hard contact can ever occur under reasonable circumstances.

Those skilled in the art will recognize that there are many equivalents to the specific embodiments described herein. For example, although only two specific designs for the entrapment zone and only one embodiment for the means for providing breakaway torque under exceptional circumstances have been enumerated, a number of other configurations are possible. Such equivalents are intended to be encompassed within the scope of the following claims.

Industrial Applicability

This invention has industrial applicability in clinical laboratories, blood banks, etc., in the separation of blood into two or more components.

**Claims**

1. A centrifuge for processing liquids and having a rotatable bowl (12), stationary elements (14, 18 etc), means (19, 20) respectively for admitting and discharging liquid to and from the centrifuge, and a dynamic seal between rotary and stationary parts of the centrifuge, the seal comprising a rotatable lower ring (40) and a normally motionless upper ring (42), characterised in that radially inwardly of their area of sealing contact (44) the rings (40, 42 or 50) are formed to provide a space (46) therebetween wherein solid particulate matter generated at the area of contact is entrapped in use and caused to settle upon the lower ring (40) which, by its rotation, sweeps the solid matter in a radially outward direction into the area of contact for ingestion therein and ultimate expulsion radially outwards from the seal.

2. A centrifuge according to claim 1, characterised in that the lower rotary ring (40) has a flat surface confronting the normally motionless upper ring (42 or 50) which has a recessed portion radially inwardly of the area of sealing contact (44) to provide the said space (46).

3. A centrifuge according to claim 2, characterised in that the upper ring (50) has a plurality of adjacent grooves (52, 54, 56) in its lower face constituting the recessed portion.

4. A centrifuge according to any of claims 1 to 3, characterised in that the lower rotary ring (40) has a smaller inside diameter than the normally motionless ring (42) or 50) to provide an inwardly-extending shoulder to catch solid matter and accelerate it centrifugally toward the area of sealing contact (44).

5. A centrifuge according to any of claims 1 to 4, characterised by the normally motionless ring (42) or 50) having a stepped configuration with a lowermost portion (44) to make sealing contact with the lower rotary ring (40) and a recessed portion radially inwardly thereof, a corner (48) between the said portions being rounded to facilitate entry of solid matter into the area of sealing contact from the space (46).

6. A centrifuge according to any of claims 1 to 5, characterised in that the lower rotary ring (40) has an outwardly extended skirt of flange (90) to aid in dissipating heat and is made from a good heat-conducting material.

7. A centrifuge according to any of claims 1 to 6, further characterised by a resilient diaphragm (68) extending between the normally motionless ring (42 or 50) and a stationary part of the centrifuge, and providing a second liquid tight seal.

8. A centrifuge according to claim 7, characterised by means for providing breakaway torque between the seal rings (40, 42 or 50) without significant concomitant buildup of torque on the resilient diaphragm (68).

9. A centrifuge according to claim 8 characterised in that the said means for providing breakaway torque comprises tabs (112, 114, 116) on the periphery of the normally motionless ring (42 or 50) and recesses (118, 120 122) for the tabs in a staionary centrifuge part (124), the tabs being free from contact with the centrifuge part (124) in normal operation but being movable to abut ends of the recesses (118, 120, 122) in the event that additional torque is required for breakaway, movement of the tabs being caused by a limited rotation of the normally motionless seal ring (42 or 50).

10. A centrifuge according to any of claims 7 to 9, characterised by a stationary shield (102, 124) and by a flange on the diaphragm (68) extending beyond the periphery of the normally motionless seal ring (42 or 50) for contact with the shield (102, 124) on the event of misalignment occurring between rotatable and stationary parts of the centrifuge.

11. A centrifuge according to claim 10 characterised by the protective shield (102, 124) extending around the rotary seal (40).

12. A centrifuge according to any of claims 1 to 11, further characterised by a cylindrical rotatable shield (66) positioned inwardly of the dynamic seal (40, 42 or 50) and projecting above the plane of the rubbing surfaces of the seal to deflect solid matter impinging thereon towards the area of contact between the seal rings.

## Revendications

1. Centrifugeuse pour traiter des liquides, comprenant une chloche rotative (12), des éléments fixes (14, 18 etc), des moyens (19, 20) pour admettre et pour évacuer respectivement un liquide dans et hors de la centrifugeuse, et un joint dynamique entre les parties fixes et rotatives de la centrifugeuse, ce joint comprenant une bague inférieure rotative (40) et une bague supérieure (42) normalement fixe, caractérisée en ce que, radialement vers l'intérieur de leur zone de contact étanche (44), les bagues (40, 42 ou 50) sont agencées pour délimiter un espace (46) entre elles dans lequel de la matière en particules solides générée à la zone de contact est emprisonnée durant l'utilisation et se dépose sur la bague inférieure (40) laquelle, par sa rotation, balaye la matière solide dans une direction radialement extérieure pour la faire entrer à l'intérieur de la zone de contact afin de finalement l'expulser radialement vers l'extérieur du joint.

2. Centrifugeuse selon la revendication 1, caractérisée en ce que la bague rotative inférieure (40) présente une surface plane située en regard de la bague supérieure (42 ou 50) normalement fixe qui comporte une partie évidée radialement vers l'intérieur de la zone de contact étanche (44) afin de délimiter ledit espace (46).

3. Centrifugeuse selon la revendication 2, caractérisée en ce que la bague supérieure (50) présente une pluralité de gorges adjacentes (52, 54, 56) dans sa face inférieure constituant la partie évidée.

4. Centrifugeuse selon l'une quelconque des revendication 1 à 3, caractérisée en ce que la bague rotative inférieure (40) présente un diamètre intérieur plus petit que la bague (42 ou 50) normalement fixe afin de constituer un épaulement s'étendant ver l'intérieur pour attraper la matière solide et l'accélérer par centrifugation vers la zone de contact étanche (44).

5. Centrifugeuse selon l'une quelconque des revendications 1 à 4, caractérisée par le fait que la bague (42 ou 50) normalement fixe présente une configuration étagée ou en gradin avec une partie la plus basse (44) pour assurer un contact étanche avec la bague rotative inférieure (40) et une partie évidée radialement vers l'intérieur de ladite bague, un coin (48) entre lesdites parties étant arrondi pour faciliter l'entrée de la matière solide à l'intérieur de la zone de contact étanche à partir de l'espace (46).

6. Centrifugeuse selon l'une quelconque des revendications 1 à 5, caractérisée en ce que la bague rotative inférieure (40) comporte une jupe ou bride (90) s'étendant vers l'extérieur pour faciliter la dissipation de chaleur, et est réalisée en une matière bonne conductrice de la chaleur.

7. Centrifugeuse selon l'une quelconque des revendications 1 à 6, caractérisée en ce qu'elle comprend un diaphragme élastique (68) s'étendant entre la bague (42 ou 50) normalement fixe et une partie fixe de la centrifugeuse, et qui constitue un joint secondaire étanche au liquide.

8. Centrifugeuse selon la revendication 7, caractérisée en ce qu'elle comporte des moyens pour assurer un couple de démarrage entre les bagues (40, 42 ou 50) du joint sans augmentation concomitante importante du couple sur le diaphragme élastique (68).

9. Centrifugeuse selon la revendication 8, caractérisée en ce que les moyens pour assurer le couple de démarrage comprennent des ergots (112, 114, 116) sur la périphérie de la bague (42 ou 50) normalement immobile, et des encoches (118, 120, 122) pour les ergots dans une partie fixe (124) de la centrifugeuse, les ergots n'étant pas en contact avec la partie fixe (124) en fonctionnement normal de la centrifugeuse mais pouvant être déplacés pour abouter les extrémités des encoches (118, 120, 122) dans le cas où un couple additionnel est nécessaire au démarrage, le déplacement des ergots étant provoqué par une rotation limitée de la bague d'étanchéité (42 ou 50) normalement immobile.

10. Centrifugeuse selon l'une des revendications 7 à 9, caractérisée en ce qu'elle comprend un bouclier fixe (102, 124) et une bride du diaphragme (68) s'étendant au-delà de la périphérie de la bague d'étanchéité (42 ou 50) normalement immobile, afin d'être en contact avec la bouclier (102, 124) au cas où il se produirait un décalage entre les parties fixes et rotatives de la centrifugeuse.

11. Centrifugeuse selon la revendication 10, caractérisée en ce que le bouclier protecteur (102, 124) s'étend autour de la bague rotative (40).

12. Centrifugeuse selon l'une quelconque des revendications 1 à 11, caractérisée en ce qu'elle comprend un bouclier rotatif cylindrique (66) placé à l'intérieur du joint dynamique (40, 42 ou 50) et faisant saillie au-dessus du plan des surfaces en frottement du joint pour défléchir la matière solide frappant celui-ci vers la zone de contact entre les bagues d'étanchéité.

**Patentansprüche**

1. Zentrifuge zum Behandeln von Flüssigkeiten, mit einem drehbaren Gefäß (12), ortsfesten Elementen (14, 18 etc), Mitteln (19, 20) zum Zu- und Abführen von Flüssigkeit zu bzw. von der Zentrifuge und einer Berührungsdichtung zwischen drehenden und ortsfesten Teilen der Zentrifuge, die einen drehbaren unteren Ring (40) und einen normalerweise bewegungslosen oberen Ring (42) umfaßt, dadurch gekennzeichnet, daß die Ringe (40, 42 oder 50) von ihrem Dichtungsberührungsbereich (44) radial nach innen zwischen sich einen Raum (46) bilden, in dem im Berührungsbereich erzeugte Feststoffpartikel im Betrieb eingeschlossen und zum Absetzen auf den unteren Ring (40) gebracht werden, der, durch seine Drehung, den Feststoff in radial nach außen weisender Richtung in den Berührungsbereich zur Einführung in diesen und zum abschließenden Auswärtstreiben von der Dichtung in radialer Richtung abstreicht.

2. Zentrifuge nach Anspruch 1, dadurch gekennzeichnet, daß der entere Drehring (40) eine ebene Fläche aufweist, die dem normalerweise, bewegungslosen oberen Ring (42 oder 50) gegenüberliegt, der einen ausgenommenen Bereich radial nach innen von Dichtungsberührungsbereich (44) zum Ausbilden des genannten Raums (46) besitzt.

3. Zentrifuge nach Anspruch 2, dadurch gekennzeichnet, daß der obere Ring (50) eine Mehrzahl benachbarter Nuten (52, 54, 56) in seiner unteren Fläche aufweist, die den ausgenommenen Bereich bilden.

4. Zentrifuge nach einem der Ansprüche 1 bis 3, dadurch gekennzeichnet, daß der untere Drehring (40) einen kleineren Innendurchmesser als der normalerweise bewegungslose Ring (42 oder 50) aufweist, derart, daß eine sich nach innen erstreckende Schulter zum Auffangen von Feststoff und zu dessen zentrifugaler Beschleunigung zum Dichtungsberührungsbereich (44) hin gebildet ist.

5. Zentrifuge nach einem der Ansprüche 1 bis 4, dadurch gekennzeichnet, daß der normalerweise bewegungslose Ring (42 oder 50) eine abgestufte Gestalt mit einem untersten Bereich (44) zur Herbeiführung der Dichtungsberührung mit dem unteren Drehring (40) und einem von diesem radial nach innen angeordneten ausgenommenen Bereich aufweist, wobei eine Ecke (48) zwischen diesen Bereichen abgerundet ist, derart, daß der Eintritt von Feststoffen in den Dichtungsberührungsbereich aus dem Raum (46) erleichtert ist.

6. Zentrifuge nach einem der Ansprüche 1 bis 5, dadurch gekennzeichnet, daß der untere Drehring (40) einen sich nach außen erstrekkenden Mantel oder Flansch (90) zur Unterstützung der Wärmeverteilung aufweist und aus einem Material mit guter Wärmeleitfähigkeit hergestellt ist.

7. Zentrifuge nach einem der Ansprüche 1 bis 6, gekennzeichnet durch eine elastische Membran (68), die sich zwischen dem Normalerweise bewegungslosen Ring (42 oder 50) und einem ortsfesten Teil der Zentrifuge erstreckt und eine zusätzliche flüssigkeitsdichte Dichtung bildet.

8. Zentrifuge nach Anspruch 7, gekennzeichnet durch Mittel zum Erzeugen eines Abreißdrehmoments zwischen den Dichtungsringen (40, 42 oder 50) ohne einen dabei auftetenden nennenswerten Aufbau eines Drehmoments an der elastischen Membran (68).

9. Zentrifuge nach Anspruch 8, dadurch gekennzeichnet, daß die Mittel zum Erzeugen des Abreißdrehmoments Laschen (112, 114, 116) am Umfang des normalerweise bewegungslosen Ringes (42 oder 50) und Ausnehmungen (118, 120, 122) für die Laschen in einem orstfesten Zentrifugenteil (124) umfassen und daß die Laschen im normalen Betrieb außer Berührung mit dem Zentrifugenteil (124) stehen, jedoch in dem Fall, daß ein zusätzliches Drehmoment zum Losbrechen erforderlich ist, für eine Anlage an Enden der Ausnehmungen (118, 120, 122) bewegbar sind, wobei die Bewegung der Laschen durch eine begrenzte Drehung des normalerweise bewegungslosen Dichtungsrings (42 oder 50) hervorgerufen wird.

10. Zentrifuge nach einem der Ansprüche 7 bis 9, gekennzeichnet durch eine ortsfeste Abschirmung (102, 124) und durch einen Flansch an der Membran (68), der sich über den Umfang des normalerweise bewegungslosen Dichtungsrings (42 oder 50) zur Berührung mit der Abschirmung (102, 124) bei einer Fehlausrichtung zwischen drehbaren und orstfesten Teilen der Zentrifuge hinauserstreckt.

11. Zentrifuge nach Anspruch 10, dadurch gekennzeichnet, daß sich die Schutzabschirmung (102, 124) rundum die Drehdichtung (40) erstreckt.

12. Zentrifuge nach einem der Ansprüche 1 bis 11, gekennzeichnet durch eine zylindrische drehbare Abschirmung (66), die einwärts von der Berührungsdichtung (40, 42 oder 50) angeordnet ist und die Ebene der Dichtungsreibflächen überragt, derart, daß auf diese aufprallende Feststoffe zur Berührungsfläche zwischen den Dichtungsringen hin abgelenkt werden.

O O 15 288

FIG. 1.

0 015 288

FIG. 2.

FIG. 3.

FIG. 4.

FIG. 5.

FIG. 7.

0 015 288

FIG. 6.

3

**0 015 288**

*Fig. 8*

*Fig. 9*

*Fig. 10*

4